# DEMANDE DE BREVET EUROPEEN

(11) **EP 2 939 744 A1**
(43) Date de publication de la demande: **04.11.2015**
(21) Numéro de dépôt: 15305337.6
(22) Date de dépôt: 04.03.2015
(51) Int. Cl.: B01J 31/18, B01J 31/22, B01J 31/24

(54) **Nouveaux complexes cycliques à base de nickel et leur utilisation dans un procédé de transformation des olefines**

(30) Priorité: 28.04.2014 FR 1453818
(71) Demandeur: IFP Énergies nouvelles, 92852 Rueil-Malmaison Cedex (FR); Universiteit van Amsterdam, 1000 GG Amsterdam (NL)
(72) Inventeur: Boulens, Pierre, 69006 Lyon (FR); Breuil, Pierre-Alain, 69006 LYON (FR); Reek, Joost, NL-3817 BK Amersfoort (NL); Olivier-Bourbigou, Helene, 69230 SAINT GENIS-LAVAL (FR)

(57) **Abrégé**

L'invention décrit un nouveau type de complexe à base de nickel et sa méthode de préparation. L'invention concerne également l'utilisation dudit complexe dans un procédé de transformation des oléfines.

## Description

La présente invention concerne une nouvelle famille de complexes à base de nickel, et leur méthode de préparation. L'invention concerne également l'utilisation desdits complexes comme catalyseurs de réactions de transformations chimiques.

### Art antérieur :

Il est connu de préparer des complexes à base de métaux de transition pour leur application dans divers domaines de la chimie, notamment dans le domaine des transformations catalytiques tels que l'hydroformylation, l'hydrogénation, le couplage croisé, l'oligomérisation des oléfines...

La préparation de tels complexes passe par le choix du métal et des ligands adaptés. Parmi ces ligands, les ligands bidentes représentent une classe importante de ligands utilisés dans la préparation de catalyseurs à base de métaux de transition pour divers types de transformations chimiques catalytiques.

Le document EP 2 220 099 B1 décrit un système de complexes de coordination comprenant des ligands multidentes ayant la formule: R₁-SO₂-NH-P(XR₂)₂; ou R₁-SO₂-N=PH(XR₂)₂, ou R₁-SO(OH)=NP(XR₂)₂, dans lesquels X est indépendamment O, S, NH, ou une liaison; dans lequel R₁ et R₂ sont indépendamment choisis parmi un groupe alkyle substitué ou non et un groupe aryle, dans lequel au moins un équivalent de ligand est complexé à un équivalent d'un métal choisi parmi le rhodium, l'iridium, le platine, le palladium et les lanthanides. EP 2 220 099 B1 indique que le système de complexe de coordination peut être utilisé en tant que catalyseur pour l'hydroformylation, l'hydrogénation, l'hydrogénation, la polymérisation, l'isomérisation...

La demanderesse dans ses recherches a mis au point une nouvelle famille de complexes à base de nickel et leur méthode de préparation. Il a été constaté de manière surprenante que de tels complexes présentent des propriétés catalytiques intéressantes. En particulier, ces catalyseurs présentent une bonne activité dans l'oligomerisation des oléfines.

Un objectif de l'invention est de fournir une nouvelle famille de complexes à base de nickel. Un autre objectif de l'invention est de proposer un nouveau système catalytique comprenant lesdits complexes pour des réactions de transformations chimiques, en particulier pour l'oligomerisation des oléfines.

### Description détaillée de l'invention

### Complexes de nickel

Les complexes selon l'invention sont des complexes à base de nickel répondant à la formule (I) ou (II) 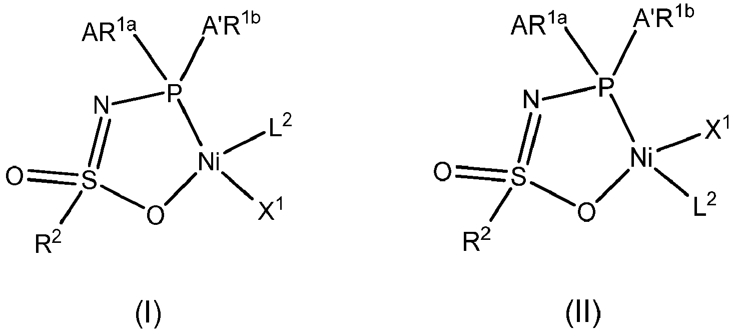 dans lesquels
- les atomes P, N, S, O constituent un fragment de ligand,
- A et A', identiques ou différents, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R¹, représentés sur les formules par R^{1a} et R^{1b}, avec R^{1a} et R^{1b} étant identiques ou différents entre eux, liés ou non entre eux, sont choisis parmi les groupements alkyle cycliques ou non, substitués ou non et contenant ou non des hétéroéléments, les groupements aromatiques, substitués ou non et contenant ou non des hétéroéléments,
- le groupement R² est choisi parmi les groupements alkyles cycliques ou non, substitués ou non et contenant ou non des hétéroéléments, les groupements aromatiques substitués ou non et contenant ou non des hétéroéléments,
- L² représente une base de Lewis,
- X¹ est un atome d'hydrogène ou un halogène ou un atome de carbone lié à ou faisant partie d'au moins un groupement alkyle cyclique ou non, insaturé ou non, substitué ou non et contenant ou non des hétéroéléments, un groupement aromatique substitué ou non et contenant ou non des hétéroéléments,
- L² et X¹ sont tels que le degré d'oxydation du nickel est respecté.

Au sens de la présente invention, on entend par « alkyle » une chaîne hydrocarbonée linéaire ou ramifiée ayant de 1 à 15 atomes de carbone et de préférence de 1 à 10. Des groupes alkyles préférés sont avantageusement choisis parmi les groupes méthyle, éthyle, propyle, isopropyle, butyle, isobutyle et tertio-butyle. Ces groupements alkyles peuvent être substitués par des hétéroéléments ou des groupes contenant des hétéroéléments tels qu'un halogénure, un alcoxy. On entend par un substituant « alcoxy », un groupe alkyl-O- dans lequel le terme alkyle a la signification donnée ci-dessus. Des exemples préférés de substituants alcoxy sont les groupes méthoxy ou éthoxy.

Par « alkyle cyclique », on entend on entend un groupe hydrocarboné monocyclique ayant un nombre de carbone supérieur à 3, de préférence entre 4 et 24, de manière plus préférée entre 6 et 12, de préférence un groupe cyclopentyle, cyclohexyle, cyclooctyle ou cyclododécyle, ou polycyclique (bi- ou tricyclique) ayant un nombre de carbone supérieur à 3, de préférence entre 4 et 18, tels que par exemple les groupes adamantyle ou norbornyle.

Par « alkyle insaturé linéaire » ou « alkyle insaturé cyclique », on entend un alkyle linéaire ou cyclique possédant au moins une insaturation, le terme alkyle et alkyle cyclique ayant la signification donnée ci-dessus.

Par « aromatique », on entend un groupe mono- ou polycyclique aromatique, de préférence mono- ou bicyclique, ayant un nombre d'atomes de carbone entre 5 et 20. Lorsque le groupe est polycyclique, c'est-à-dire qu'il comprend plus d'un noyau cyclique, les noyaux cycliques peuvent avantageusement être condensés deux à deux ou rattachés deux à deux par des liaisons σ. Le groupe aromatique selon l'invention peut contenir un hétéroélément tel que l'azote, l'oxygène ou le soufre.

Le terme ligand selon la présente invention est indifféremment utilisé pour signifier une ou plusieurs des formes limites du ligand répondant à la formule 1a), 1b) et/ou 1c): 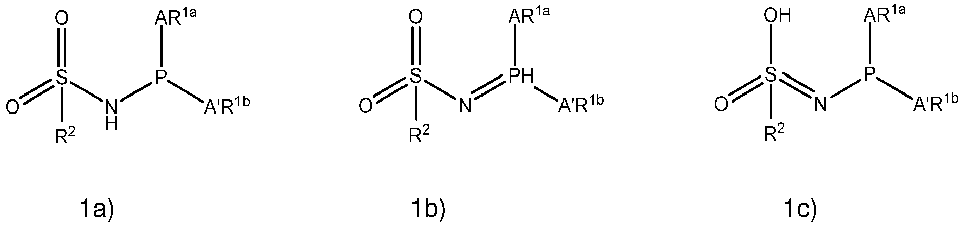 dans laquelle
- A et A', identiques ou différents, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R¹, représentés sur la formule par R^{1a} et R^{1b}, avec R^{1a} et R^{1b} étant identiques ou différents entre eux, liés ou non entre eux, sont choisis parmi les groupements alkyles cycliques ou non, substitués ou non et contenant ou non des hétéroéléments, les groupements aromatiques, substitués ou non et contenant ou non des hétéroéléments,
- le groupement R² est choisi parmi les groupements alkyles cycliques ou non, substitués ou non et contenant ou non des hétéroéléments, les groupements aromatiques substitués ou non et contenant ou non des hétéroéléments.

Les deux groupements R¹ (R^{1a} et R^{1b}) peuvent être identiques ou différents entre eux. Ces deux groupements R^{1a} et R^{1b} peuvent également être liés entre eux. Dans un tel cas, les deux groupements R^{1a} et R^{1b} peuvent correspondre à des groupements tels que le bis-phényle, le bis-naphthyle.

Les ligands selon l'invention peuvent être préparés par une réaction de condensation d'un sulfonamide, par exemple le para-*n*-butylphényl-sulfonamide et d'un halogénure de phosphine, tel que Ph₂PCl, en présence d'une base de Brönsted telle que la triéthylamine par exemple, dans un solvant. En solution, ces ligands peuvent (co)exister sous les trois formes 1a), 1b) ou 1c) décrites ci-dessus.

L² représente une base de Lewis. Au sens de la présente invention, on entend par « base de Lewis », toute entité chimique dont un constituant possède un doublet ou plus d'électrons libres ou non liants. Les bases de Lewis selon l'invention correspondent en particulier à tout ligand comprenant un atome d'oxygène, d'azote ou de phosphore possédant un doublet d'électrons libres ou non liants, ou une double liaison π capable de former avec le nickel une coordination de type η².

Le groupement L² du complexe de formule (I) ou (II) selon l'invention peut représenter une phosphine de type P(A¹R'^{1a})(A'¹R'^{1b})(A"¹R'^{1c}) ou une phosphinamine de type (R'^{1a}A¹)(R_{'}^{1b}A'¹)P-NH(R'²) ou (R'^{1a}A¹)(R'^{1b}A'¹)P-NH-S(O)₂(R'²), dans lesquelles :
- A¹, A'¹ et A"¹, identiques ou différents entre eux, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R'¹, soit R'^{1a}, R^{'1b} et R^{'1c} étant identiques ou différents entre eux, liés ou non entre eux, sont choisis parmi les groupements alkyles cycliques ou non, substitués ou non et contenant ou non des hétéroéléments, les groupements aromatiques, substitués ou non et contenant ou non des hétéroéléments,
- le groupement R'² est choisi parmi les groupements alkyles cycliques ou non, substitués ou non et contenant ou non des hétéroéléments, les groupements aromatiques substitués ou non et contenant ou non des hétéroéléments.

X¹ est un atome d'hydrogène ou un halogène ou un atome de carbone lié à ou faisant partie d'au moins un groupement alkyle cyclique ou non, insaturé ou non, substitué ou non et contenant ou non des hétéroéléments, un groupement aromatique substitué ou non et contenant ou non des hétéroéléments. Avantageusement X¹ est un atome d'hydrogène, un groupement alkyle cyclique ou non, insaturé ou non, substitué ou non et contenant ou non des hétéroéléments ou un halogène. Lorsque le groupement X¹ est un halogène, ce dernier peut être un atome de brome, de chlore, d'iode ou de fluor.

Selon l'invention, les groupements R¹, soit R^{1a} et R^{1b} étant identiques ou différents, liés ou non entre eux, et les groupements R'¹, soit R'^{1a,} R^{'1b} et R^{'1c} étant identiques ou différents, liés ou non entre eux, sont indépendamment choisis parmi les groupements alkyles comprenant 1 à 15 atomes de carbones, les groupements aromatiques comprenant 5 à 20 atomes de carbone; substitués ou non, contenant des hétéroéléments ou non.

De préférence, les groupements R¹, soit R^{1a} et R^{1b} étant identiques ou différents, liés ou non entre eux, et les groupements R'¹, soit R'^{1a,} R^{'1b} et R^{'1c} étant identiques ou différents, liés ou non entre eux, sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant ou non des hétéroéléments; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-di(trifluorométhyl)phényle, benzyle, naphthyle, bisnaphthyle, pyridyle, bisphényle, furanyle, thiophényle, substitués ou non, contenant des hétéroéléments ou non. Dans le cas où les groupements R^{1a} et R^{1b} identiques ou différents sont liés entre eux, ces groupements peuvent correspondre à des groupements tels que le bis-phényle, le bis-naphthyle. Dans le cas où les groupements R'¹ identiques ou différents sont liés entre eux, ces groupements peuvent correspondre à des groupements tels que le bis-phényle, le bis-naphthyle.

Selon l'invention, les groupements R² et les groupements R'², identiques ou différents, sont indépendamment choisis parmi les groupements alkyles comprenant 1 à 15 atomes de carbones, les groupements aromatiques comprenant 5 à 20 atomes de carbone; substitués ou non, contenant des hétéroéléments ou non.

De préférence, les groupements R² et les groupements R'², identiques ou différents, sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant des hétéroéléments ou non ; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-bis(trifluorométhyl)phényle, benzyle, naphthyle, bisnaphthyle, pyridyle, bisphényle, furanyle, thiophényle, substitués ou non, contenant des hétéroéléments ou non.

De préférence le groupement R³ est soit un atome d'hydrogène, soit un groupement alkyle.

Les complexes selon l'invention peuvent être préparés par la mise en contact d'au moins un ligand comprenant le fragment de ligand constitué par les atomes P, N, S et O répondant à la formule (1a), (1 b) ou (1 c) tel que défini selon l'invention, avec au moins un précurseur de nickel de degré d'oxydation (0), un précurseur du groupement X¹, et optionnellement une base de Lewis, La présence d'une base de Lewis est par exemple optionnelle lorsqu'un deuxième équivalent du ligand est mis en oeuvre. Dans ce cas le ligand selon l'invention joue le rôle d'une base de Lewis. Dans un mode particulier de réalisation, le groupement X¹ peut provenir du ligand lui-même.

Les complexes selon l'invention peuvent également être préparés par la mise en contact d'au moins un ligand comprenant le fragment de ligand constitué par les atomes P, N, S et O répondant à la formule (1a), (1b) ou (1c) tel que défini selon l'invention, avec au moins un précurseur de nickel de degré d'oxydation (+II) en présence d'un agent réducteur ou d'une base de Brônsted; et optionnellement une base de Lewis. La présence d'une base de Lewis est, par exemple, optionnelle lorsqu'un deuxième équivalent du ligand est mis en oeuvre. Dans ce cas le ligand selon l'invention joue le rôle d'une base de Lewis. Dans un mode particulier de réalisation, le groupement X¹ peut provenir du précurseur de nickel de degré d'oxydation (+II).

Dans le cas où un précurseur de nickel de degré d'oxydation (+II) est utilisé en présence d'un agent réducteur, on peut utiliser tout agent conduisant à la réduction du nickel connu de l'homme du métier. L'agent réducteur peut être choisi parmi NaBH₄, LiAlH₄, AlEt₃, Na, K, KC₈ et le dihydrogène.

Dans le cas où un précurseur de nickel de degré d'oxydation (+II) est utilisé en présence d'une base de Brônsted, on peut utiliser toute base de Brônsted connue de l'homme du métier. On entendra par « base de Brönsted » toute entité moléculaire ou espèce chimique correspondante capable d'accepter un proton, telle que par exemple la triéthylamine.

La température de préparation des complexes selon l'invention peut varier entre -80°C et 130°C.

La préparation des complexes selon l'invention peut être réalisée en présence ou non de solvant. De préférence, la préparation est réalisée en présence d'un solvant. Le solvant de préparation peut être choisi parmi les solvants organiques et en particulier parmi les éthers, les alcools, les solvants chlorés et les hydrocarbures saturés, insaturés, aromatiques ou non, cycliques ou non. De préférence, le solvant est choisi parmi l'hexane, le cyclohexane, le méthylecyclohexane, l'heptane, le butane ou l'isobutane, les monooléfines ou dioléfines comportant de préférence 4 à 20 atomes de carbone, le cycloocta-1,5-diène, le benzène, le toluène, l'ortho-xylène, le mésitylène, l'éthylbenzène, le dichlorométhane, le chlorobenzène, le méthanol, l'éthanol, purs ou en mélange, et les liquides ioniques. Dans le cas où le solvant est un liquide ionique, il est avantageusement choisi parmi les liquides ioniques décrits dans les brevets US 6,951,831 B2 et FR 2895406 B1.

Les complexes selon l'invention comprennent un nickel de degré d'oxydation (+I) ou (+II), de préférence un nickel de degré d'oxydation (+II). Les complexes selon l'invention peuvent également former des agrégats multi-nucléaires.

Lorsque le précurseur de nickel est de degré d'oxydation (0), ce dernier peut être choisi parmi le nickel(0) bis(cycloocta-1,5-diène), le nickel(0) bis(cycloocta-1,3-diène), le nickel(0) bis(cyclooctatétraène), le nickel(0) bis(cycloocta-1,3,7-triène), le bis(o-tolylphosphito)nickel(0)(éthylène), le nickel(0) tétrakis (triphénylphosphite), le nickel(0) tetrakis(triphenylphosphine), et le nickel (0) bis(éthylène), pris seul ou en mélange. Lesdits précurseurs de nickel peuvent éventuellement être complexés à des bases de Lewis.

Lorsque le précurseur de nickel est de degré d'oxydation (+II), ce dernier peut être choisi parmi le chlorure de nickel(II), le chlorure de nickel(II)(diméthoxyéthane), le bromure de nickel(II), le bromure de nickel(II)(diméthoxyéthane), le fluorure de nickel(II), l'iodure de nickel(II), le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II) tel que par exemple le 2-éthylhexanoate, les phénates de nickel(II), l'acétate de nickel(II), le trifluoroacétate de nickel(II), le triflate de nickel(II), l'acétylacétonate de nickel(II), l'hexafluoroacétylacétonate de nickel(II), le chlorure de allylnickel(II), le bromure de allylnickel(II), le dimère du chlorure de methallylnickel(II), l'hexafluorophosphate de allylnickel(II), l'hexafluorophosphate de methallylnickel(II), le biscyclopentadienyle de nickel(II), le bisallyl de nickel(II) et le bisméthallyl nickel(II); sous leur forme hydratée ou non, pris seul ou en mélange. Lesdits précurseurs de nickel peuvent éventuellement être complexés à des bases de Lewis.

Nous présentons ci-dessous quelques exemples de complexes de formule (I) ou (II) selon l'invention ainsi que les conditions opératoires au moyen desquelles ils ont été obtenus. Ces exemples sont à titre illustratif et ne sont en aucun cas limitatifs de la portée de l'invention.

Dans le schéma 1, un complexe selon l'invention répondant à la formule (I) est illustré. L'addition de 2 équivalents du ligand **L1** représenté sous la forme limite 1b) à un équivalent de NiBr₂(DME)₂ et de la triéthylamine dans du benzène conduit au bout de 16 heures à 60°C au complexe **C1.** Dans ce complexe, les atomes de phosphore sont en position *cis* et X¹ correspond à un atome de brome. La structure du complexe a été caractérisée par diffraction aux rayons X (DRX).

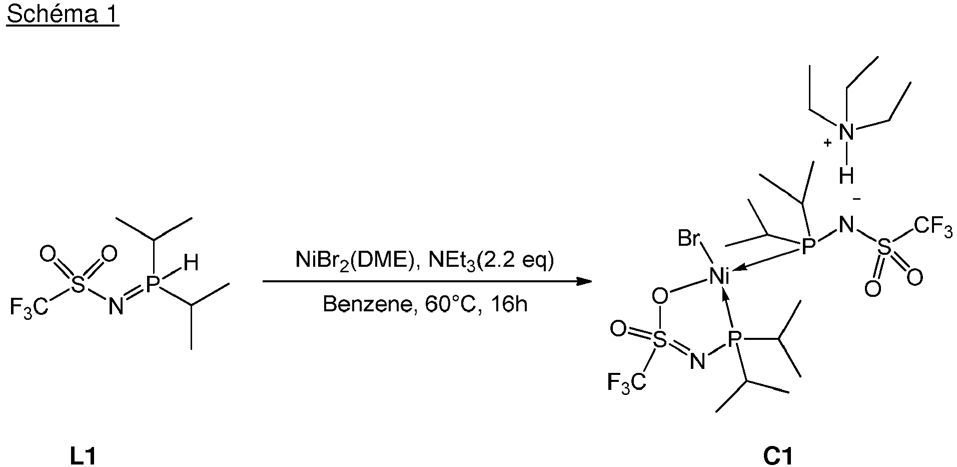

Trois complexes selon l'invention répondant à la formule (II) sont illustrés dans le schéma 2. L'addition d'un équivalent du ligand **L1** ou **L3** représentés sous la forme limite 1b) et d'un équivalent de tricyclohexylphosphine à un équivalent de nickel(0) bis(cycloocta-1,5-diène) dans du toluène conduit respectivement au bout de 3 heures et 16 heures à 60°C aux complexes **C2** ou **C3.** L'addition d'un équivalent du ligand **L4** représenté sous la forme limite 1b) et d'un équivalent de tricyclohexylphosphine à un équivalent de nickel(0) bis(cycloocta-1,5-diène) (Ni(COD)₂) dans du toluène conduit au bout de 3 heures à 90°C au complexe **C4.**

Dans ces complexes, les atomes de phosphore sont en position *trans* et X¹ correspond à un atome d'hydrogène. Les complexes sont caractérisés en RMN ³¹P par deux doublets dédoublés avec une grande constante de couplage J_{PP} de l'ordre de 230 Hz et deux plus petites constantes de couplage J_{PH} correspondant aux couplage phosphore hydrure en cis.

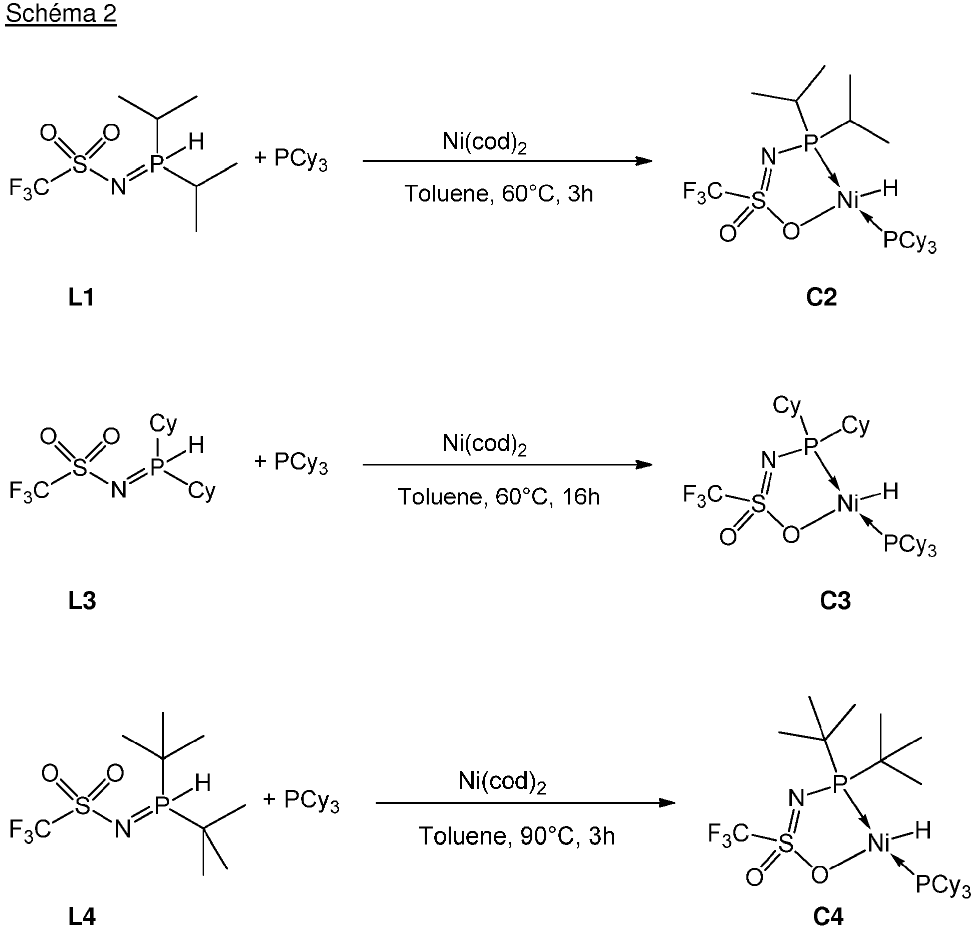

Dans le schéma 3, un complexe selon l'invention répondant à la formule (II) est illustré. Le bullage d'éthylène, pendant 3h à pression atmosphérique dans un mélange formé par un équivalent de ligand **L1,** de PCy₃ et un équivalent de Ni(COD)₂ conduit à la formation du complexe **C5.**

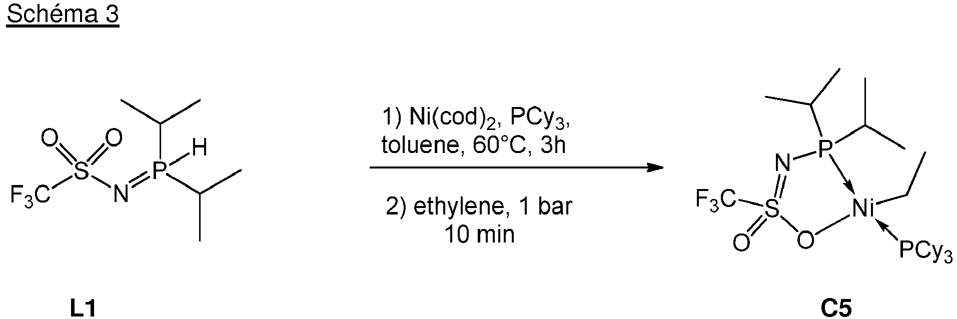

D'une manière préférée, les complexes selon l'invention répondent aux formules (I) ou (II) suivantes : 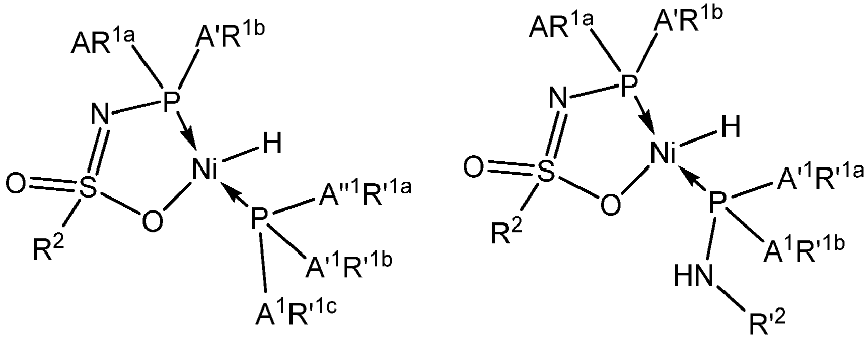 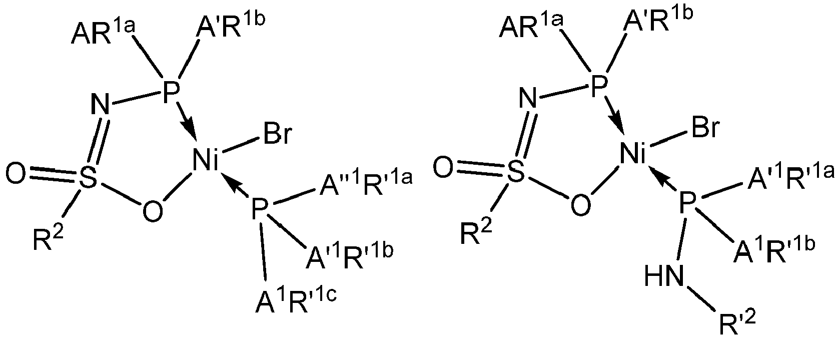 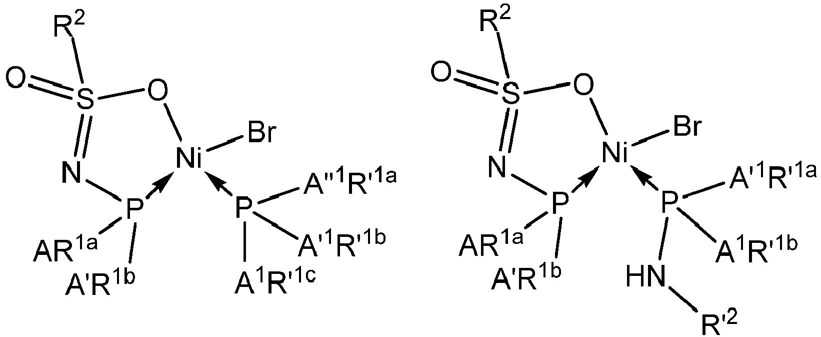 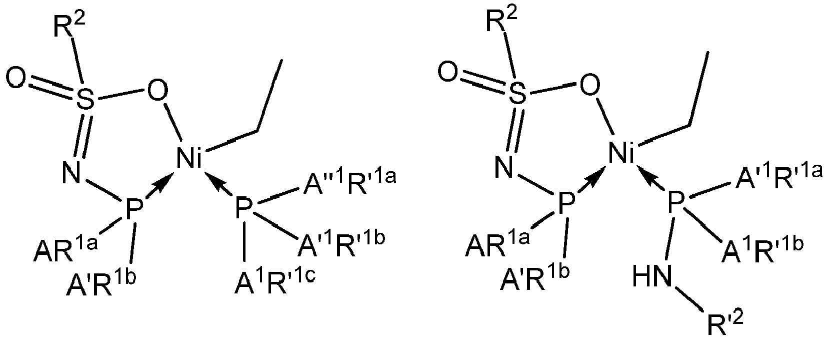 dans lesquelles le nickel est de degré d'oxydation (+II), et A, A', A¹, A'¹, A"¹, R^{1a}, R^{1b}, R'^{1a}, R'^{1b}, R'^{1c}, R² et R'² répondent aux spécifications selon l'invention.

Une liste non exhaustive de ligands pouvant convenir à la préparation des complexes selon l'invention est représentée ci-dessous. Les ligands sont représentés sous leurs formes limites 1a) et 1b).

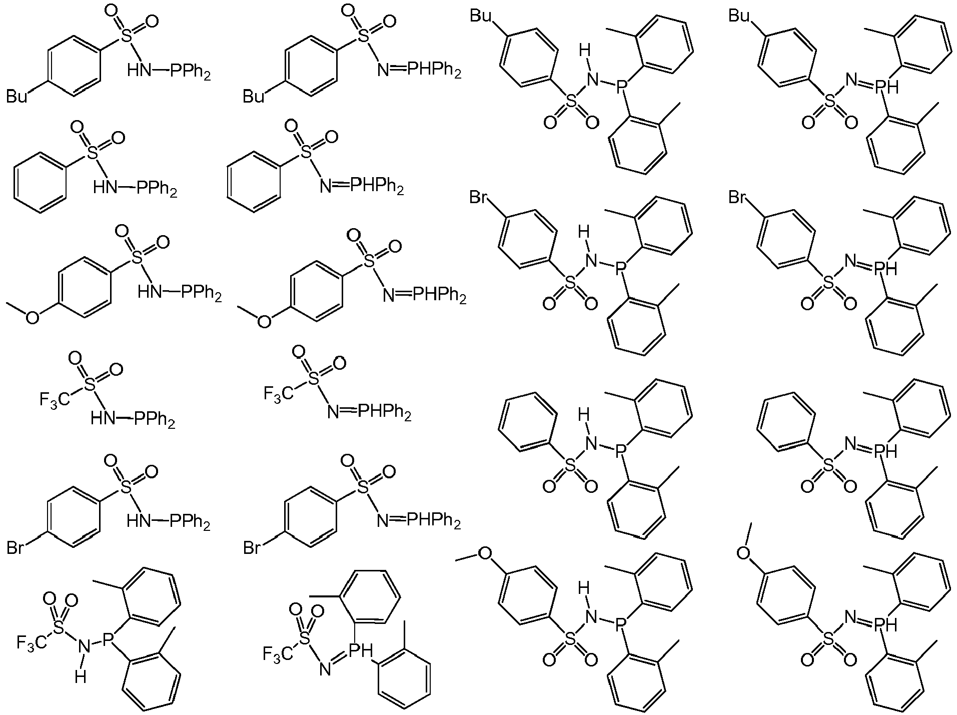

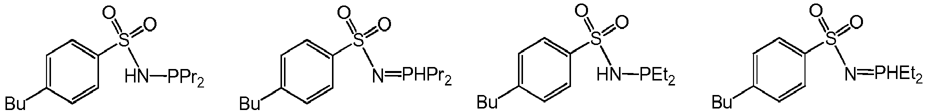

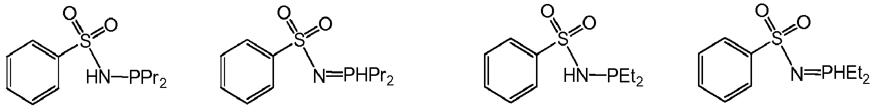

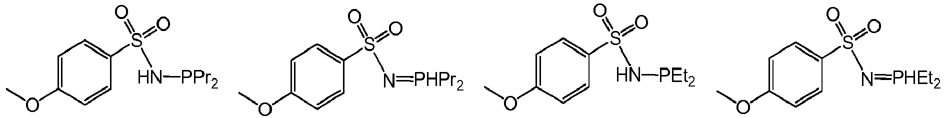

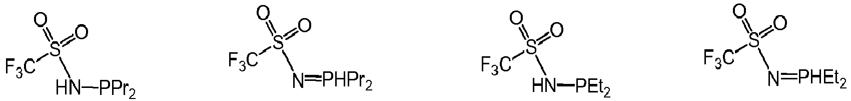

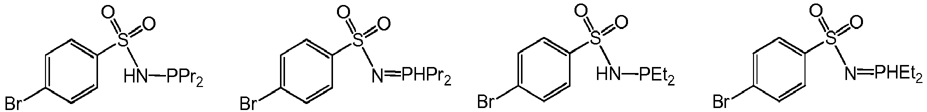

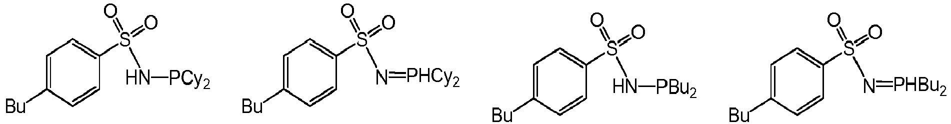

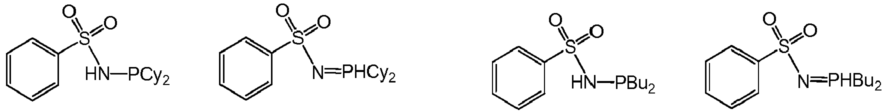

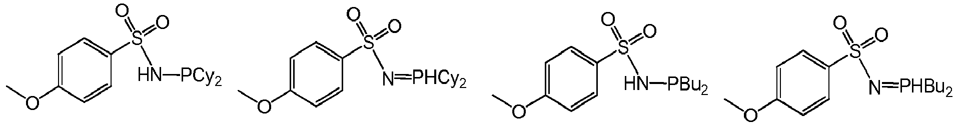

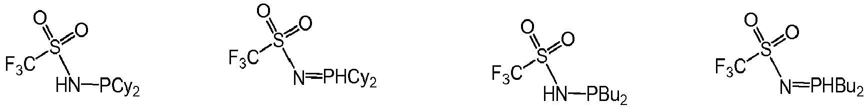

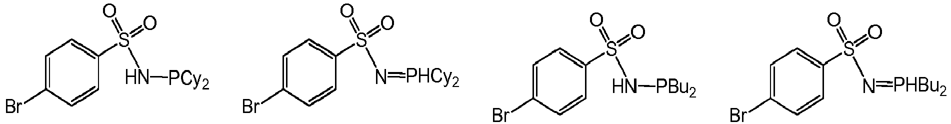

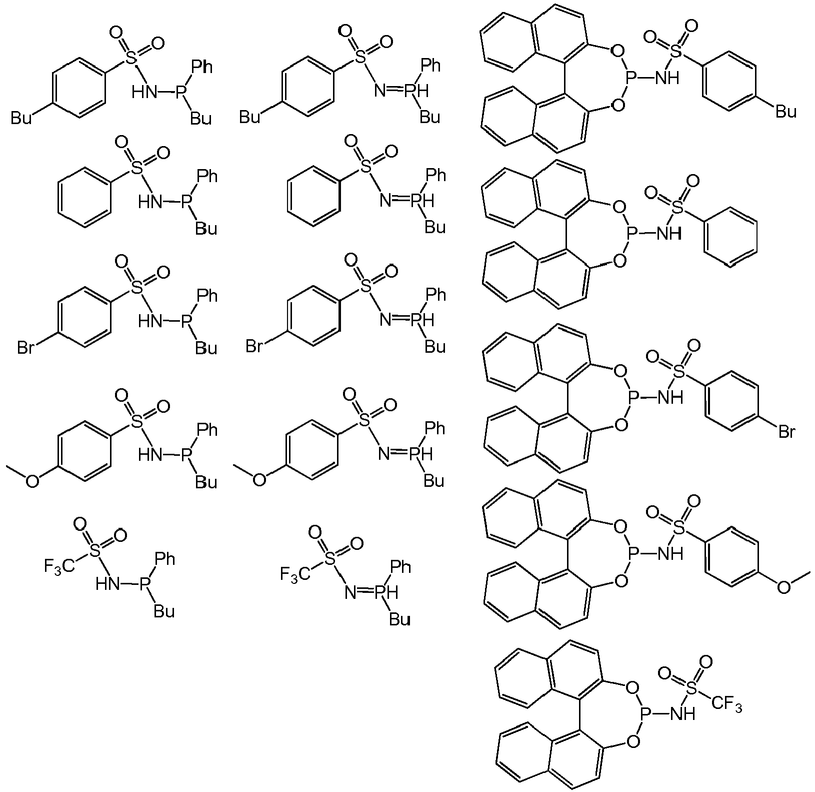

### Utilisation des complexes de formule (I) ou (II) dans une réaction de transformation chimique

Les complexes à base de nickel de formule (I) ou (II) selon l'invention peuvent être utilisés comme catalyseur dans une réaction de transformation chimique, telle que la réaction d'hydrogénation, d'hydroformylation, de couplage croisé ou d'oligomerisation des oléfines. En particulier, ces complexes sont utilisés dans un procédé d'oligomerisation d'une charge d'oléfines ayant avantageusement 2 à 10 atomes de carbone.

Les complexes de nickel de formule (I) ou (II) selon l'invention peuvent être utilisés sous forme de composition catalytique, en mélange avec un composé appelé agent activateur. Ledit agent activateur est avantageusement choisi dans le groupe formé par les composés tris(hydrocarbyl)aluminium, les composés chlorés ou bromés d'hydrocarbylaluminium, les halogénures d'aluminium, les aluminoxanes, les composés organo-borés, les composés organiques susceptibles de donner ou de capter un proton, pris seuls ou en mélange.

Les tris(hydrocarbyl)aluminium, les composés chlorés ou bromés d'hydrocarbylaluminium et les halogénures d'aluminium répondent de préférence à la formule générale AlₓR_{y}W_{z} dans laquelle R représente un radical hydrocarboné monovalent contenant par exemple jusqu'à 12 atomes de carbone tel que alkyle, aryle, aralkyle, alkaryle ou cycloalkyle, W représente un atome d'halogène choisi par exemple parmi le chlore et le brome, W étant de préférence un atome de chlore, x prend une valeur de 1 à 2, y et z prennent une valeur de 0 à 3. Comme exemples de tels composés, on peut mentionner le sesquichlorure d'éthylaluminium (Et₃Al₂Cl₃), le dichlorure de méthylaluminium (MeAlCl₂), le dichlorure d'éthylaluminium (EtAlCl₂), le dichlorure d'isobutylaluminium (iBuAlCl₂), le chlorure de diéthylaluminium (Et₂AlCl), le triméthylaluminium, le tributylaluminium, le tri-n-octylaluminium et le triéthylaluminium (AlEt₃).

Dans le cas où ledit agent activateur est choisi parmi les aluminoxanes, ledit agent activateur est avantageusement choisi parmi le méthylaluminoxane (MAO), l'éthylaluminoxane et les méthylaluminoxanes modifiés (MMAO). Ces agents activateurs peuvent être utilisés seuls ou en mélange.

De préférence, ledit agent activateur C est choisi parmi le dichloroéthylaluminium (EtAlCl₂) et le méthylaluminoxane (MAO).

Dans le cas où ledit agent activateur est choisi parmi les composés organoborés, ledit agent activateur est de préférence choisi parmi les acides de Lewis de type tris(aryl)borane tels que le tris(perfluorophényl)borane, le tris(3,5-bis(trifluorométhyl)phényl)borane, le tris(2,3,4,6-tétrafluorophényl)borane, le tris(perfluoronaphtyl)borane, le tris(perfluobiphényl)borane et leurs dérivés et les (aryl)borates associés à un cation triphénylcarbénium ou à un cation ammonium trisubstitué tels que le triphénylcarbenium tétrakis(perfluorophényl)borate, le N,N-diméthylanilinium tétrakis(perfluorophényl)borate, le N,N-diéthylanilinium tétrakis(3,5-bis(trifluorométhyl)phényl)borate, le triphénylcarbenium tétrakis(3,5-bis(trifluorométhyl)phényl)borate.

Dans le cas où ledit agent activateur est choisi parmi les composés organiques susceptibles de donner un proton, ledit agent activateur est de préférence choisi parmi les acides de formule HY dans lequel Y représente un anion.

Dans le cas où ledit agent activateur est choisi parmi les composés organiques susceptibles de capter un proton, ledit agent activateur est de préférence choisi parmi les bases de Brönsted.

Le solvant du procédé d'oligomérisation peut être choisi parmi les solvants organiques et de préférence parmi les éthers, les alcools, les solvants chlorés et les hydrocarbures saturés, insaturés, aromatiques ou non, cycliques ou non. En particulier, ledit solvant est choisi parmi l'hexane, le cyclohexane, le méthylecyclohexane, l'heptane, le butane ou l'isobutane, les monooléfines ou dioléfines comportant de préférence 4 à 20 atomes de carbone, le cycloocta-1,5-diène, le benzène, le toluène, l'ortho-xylène, le mésitylène, l'éthylbenzène, le dichlorométhane, le chlorobenzène, le méthanol, l'éthanol, purs ou en mélange, et les liquides ioniques. Dans le cas où ledit solvant de réaction est un liquide ionique, il est avantageusement choisi parmi les liquides ioniques décrits dans les brevets US 6,951,831 B2 et FR 2895406 B1.

L'oligomérisation est définie comme la transformation d'une unité monomère en un composé ou mélange de composés de formule générale CpH2p avec 4 ≤ p ≤ 80, de préférence avec 4 ≤ p ≤ 50, de manière préférée avec 4 ≤ p ≤ 26 et de manière plus préférée avec 4 ≤ p ≤ 14.

Les oléfines utilisées dans le procédé d'oligomérisation sont des oléfines comportant de 2 à 10 atomes de carbone. De préférence, lesdites oléfines sont choisies parmi l'éthylène, le propylène, les n-butènes et les n-pentènes, seules ou en mélange, pures ou diluées.

Dans le cas où lesdites oléfines sont diluées, lesdites oléfines sont diluées par un ou plusieurs alcane(s), tels qu'on les trouve dans des « coupes » issues des procédés de raffinage du pétrole, comme le craquage catalytique ou le craquage à la vapeur.

De manière préférée, l'oléfine utilisée dans le procédé d'oligomérisation est l'éthylène.

Lesdites oléfines peuvent venir de ressources non fossiles telles que la biomasse. Par exemple, les oléfines utilisées dans le procédé d'oligomérisation selon l'invention peuvent être produites à partir d'alcools, et en particulier par déshydratation des alcools.

La concentration du nickel dans la solution catalytique est avantageusement comprise entre 1.10⁻⁸ et 1 mol/L, et de préférence entre 1.10⁻⁶ et 1.10⁻² mol/L.

Le procédé d'oligomérisation opère avantageusement à une pression totale comprise entre la pression atmosphérique et 20 MPa, de préférence entre 0,1 et 8 MPa, et à une température comprise entre -40 et +250°C, de préférence entre -20°C et 150°C.

La chaleur engendrée par la réaction peut être éliminée par tous les moyens connus de l'homme du métier.

Le procédé d'oligomérisation peut être conduit en système fermé, en système semi-ouvert ou en continu, avec un ou plusieurs étages de réaction. Une vigoureuse agitation est avantageusement mise en oeuvre pour assurer un bon contact entre le ou les réactifs et le système catalytique.

Le procédé d'oligomérisation peut être mis en oeuvre en discontinu. Dans ce cas, un volume choisi de la solution comprenant le complexe selon l'invention est introduit dans un réacteur muni des dispositifs habituels d'agitation, de chauffage et de refroidissement.

Le procédé d'oligomerisation peut également être mis en oeuvre en continu. Dans ce cas, la solution comprenant le complexe selon l'invention est injectée en même temps que l'oléfine dans un réacteur agité par les moyens mécaniques classiques ou par une recirculation extérieure, et maintenue à la température souhaitée.

La composition catalytique est détruite par tout moyen habituel connu par l'homme du métier, puis les produits de la réaction ainsi que le solvant sont séparés, par exemple par distillation. L'oléfine qui n'a pas été transformée peut être recyclée dans le réacteur.

Le procédé selon l'invention peut être mis en oeuvre dans un réacteur à un ou plusieurs étages de réaction en série, la charge oléfinique et/ou la composition catalytique au préalable pré-conditionnée étant introduites en continu, soit dans le premier étage, soit dans le premier et un autre quelconque des étages. A la sortie du réacteur, la composition catalytique peut être désactivée, par exemple par injection d'ammoniac et/ou d'une solution aqueuse de soude et/ou d'une solution aqueuse d'acide sulfurique. Les oléfines non converties et les alcanes éventuellement présents dans la charge sont ensuite séparés des oligomères par distillation.

Les produits du présent procédé peuvent trouver une application par exemple comme composants de carburants pour automobiles, comme charges dans un procédé d'hydroformylation pour la synthèse d'aldéhydes et d'alcools, comme composants pour l'industrie chimique, pharmaceutique ou la parfumerie et/ou comme charges dans un procédé de métathèse pour la synthèse de propylène par exemple.

Les exemples suivants illustrent l'invention sans en limiter la portée. La notation Cy représente le groupement cyclohéxyle.

### Exemples

### Exemple 1 : Synthèses des ligands et des complexes

### Synthèse des ligands L1, L3 et L4.

La synthèse des ligands **L1, L3** et **L4** a été réalisée selon la méthode décrite dans la littérature : F. G. Terrade, Eur. J. Inorg. Chem. 2014, 1826-1835.

### Synthèse du complexe C1

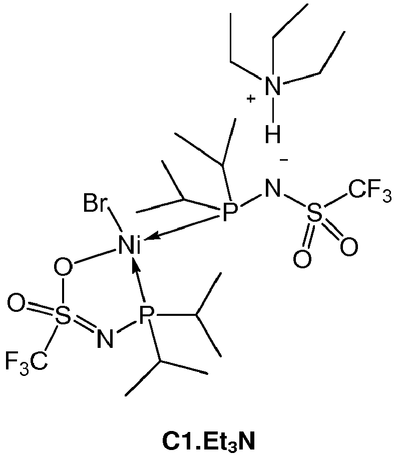

Le ligand **L1** F₃C-SO₂-N=P(*ⁱPr*)₂H (82 mg, 0.31 mmol, 2.2 eq.), du NiBr₂ (DME) (43 mg, 0.14 mmol, 1 eq.) et de la triéthylamine (100 µL, 0.74 mmol, 5.3 eq) sont suspendus dans un Schlenk avec 2 mL de benzène et agités 10 minutes à température ambiante. Puis, le mélange est chauffé à 60°C pendant 16 heures (h) puis le solvant est évaporé. La poudre rouge obtenue est lavée avec du pentane. Des cristaux sont obtenus par diffusion de pentane dans une solution de toluène.

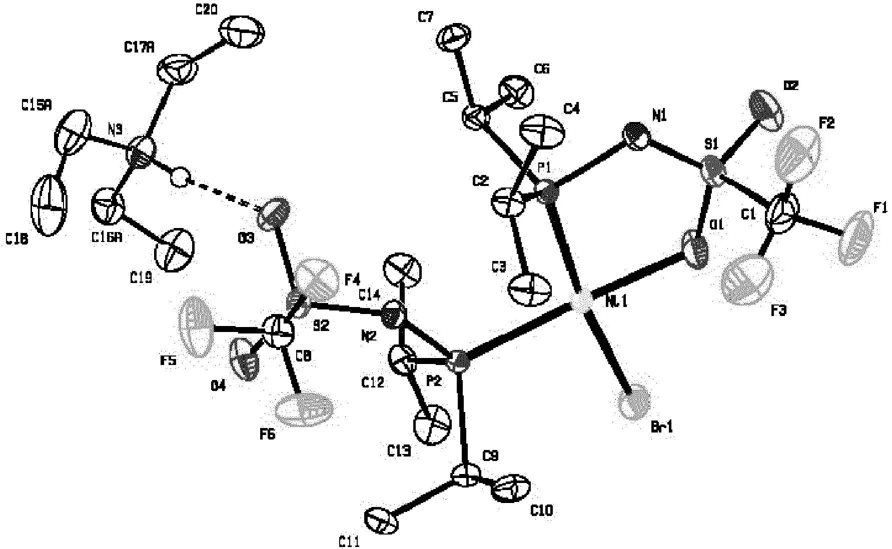

### Synthèse du complexe C2

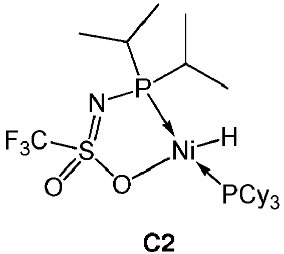

Le ligand **L1** (F₃C-SO₂-N=P(iPr)₂H, 796 mg, 3 mmol, 1eq.), du Ni(COD)₂ (825 mg, 3 mol, 1 eq.) et de la tricyclohexylphosphine (840 mg, 3 mmol, 1 eq.) sont solubilisés dans 30 mL de toluène. La solution est agitée jusqu'à dissolution des composants puis chauffée à 60°C pendant 3h. Le solvant est évaporé sous pression réduite pour donner une poudre. Après trituration et lavage dans du pentane (3 x 10 mL), on obtient une poudre jaune qui séchée sous vide correspond au produit isolé : 694 mg, 38%.
³¹P NMR (C₆D₆): 36.5 (dd, *²J_{PP}* =232 Hz et 28.6 Hz) ; 103.0 (dd, *²J_{PP}* =233 Hz et J_{PH}= 72.9 Hz)

### Synthèse du complexe C3

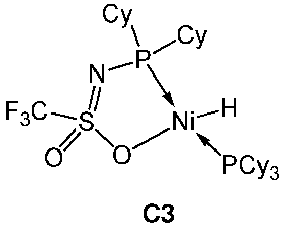

Le ligand **L3** (F₃C-SO₂-N=P(Cy)₂H, 205 mg, 0.5 mmol, 1 eq.), du Ni(COD)₂ (412 mg, 1.5 mmol, 1 eq.) et de la tricyclohexylphosphine (420 mg, 1.5 mmol, 1 eq.) sont solubilisés dans 20 mL de toluène. La solution est agitée jusqu'à dissolution des composants puis chauffée à 60°C pendant 16h. Le sol vant est évaporé sous pression réduite pour donne une poudre. Après trituration et lavage dans le pentane (3 x 10 mL) on obtient une poudre jaune (isolé: 465 mg, 45%). ³¹P NMR (C₆D₆): 36.6 (dd, *²J_{PP}* =236 Hz and 67 Hz) ; 78.6 (dd, *²J_{PP}* =237 Hz and J_{PH}= 82 Hz).

### Synthèse du complexe C4

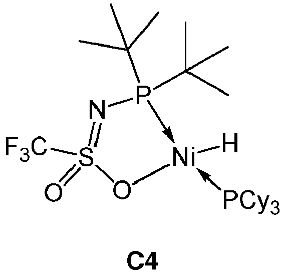

Le ligand **L4** (F₃C-SO₂-N=P(*^{t}*Bu)₂H, 58 mg, 0.2 mmol, 1 eq.), de la tricyclohexylphosphine (56 mg, 0.2 mmol, 1 eq.) et du Ni(COD)₂ (55 mg, 0.2 mmol, 1 eq.) sont placés dans un Schlenk dans 5 mL de toluène. Le mélange est chauffé et agité à 90°C pendant 3h. Le solvant est ensuite évaporé pour conduire à un solide. Ce solide est trituré puis lavé avec du pentane (3x 5 mL) pour conduire à un solide jaune. Le produit est caractérisé par RMN ³¹P(C₆D₆) : 40.4 ppm (dd, *²J_{PP}*=235 Hz et *²J_{PH}*=69 Hz) ; 120.7 (*²J_{PP}*=235 Hz et *²J_{PH}*=68 Hz).

### Synthèse du complexe C5

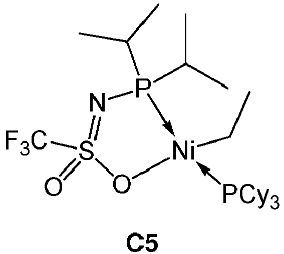

Le ligand **L3** (F₃C-SO₂-N=P(iPr)₂H, 205 mg, 0.5 mmol, 1 eq.), du Ni(COD)₂ (412 mg, 1.5 mmol, 1 eq.) et de la tricyclohexylphosphine (420 mg, 1.5 mmol, 1 eq.) sont dissous dans 20 mL de toluène. La solution est agitée jusqu'à dissolution des composants puis chauffée à 60°C pendant 3h. Un flux d'éthylène est passé dans cette solution jusqu'à ce que la couleur devienne claire (10 minutes à température ambiante, sous agitation et 1 bar d'éthylène). On obtient alors le complexe **C5.** Le produit est caractérisé par RMN ³¹P(C₆D₆) : 89.6 (d, *²J_{PP}*=245 Hz) ; 18.7 (d, *²J_{PP}* =243 Hz).

### Exemple 2: oligomérisation de l'éthylène

La réaction d'oligomérisation de l'éthylène a été évaluée avec les complexes **C2** et **C3** (10 µmoles). Les résultats obtenus sont reportés dans le tableau 1.

Le réacteur de 250 mL est séché sous vide à 130°C pendant 2 heures puis pressurisé avec 0,5 MPa d'éthylène. La température est abaissée à 20°C, puis la surpression d'éthylène est évacuée pour atteindre 0,1 MPa. Le solvant est ajouté (45 mL de toluène), et la consigne de température interne est fixée (40°C). Une fois la température interne stabilisée, le complexe est introduit (10 µmol dans 5 mL de toluène). Ensuite le réacteur est pressurisé avec de l'éthylène à 3 MPa. L'agitation (1000 tr/min) est mise en route (t=0). Après le temps de réaction défini, le mélange est refroidi à 30°C sous agitation, le réacteur est dépressurisé et les phases liquides et gaz sont analysées par chromatographie en phase gazeuse (GC).

La productivité (g_{oligo}/(g_{Ni}.h) est exprimée en masse d'oligomères produits (en gramme) par masse de nickel mis en oeuvre et par heure.

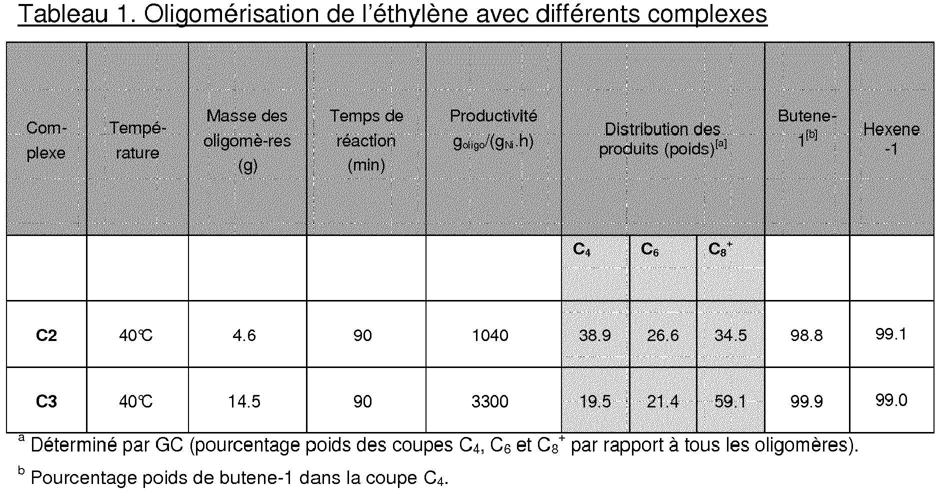

Les exemples ci-dessus démontrent que les complexes selon l'invention présentent une bonne activité pour l'oligomérisation de l'éthylène.

## Revendications

1. Complexe à base de nickel répondant à la formule (I) ou à la formule (II) 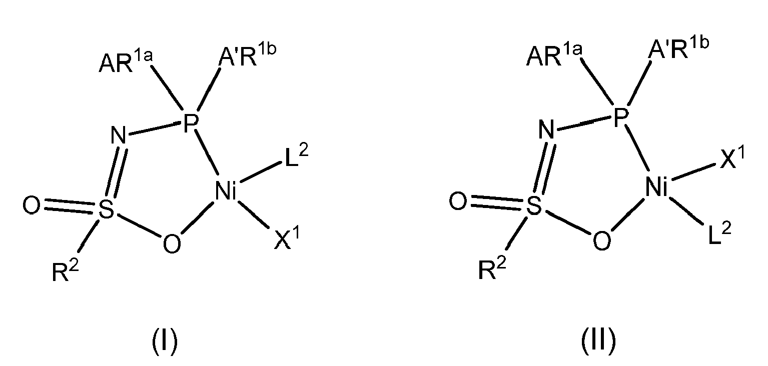 dans lequel
- les atomes P, N, S, O constituent un fragment de ligand,
- A et A', identiques ou différents, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R¹, représentés sur les formules par R^{1a} et R^{1b}, avec R^{1a} et R^{1b} étant identiques ou différents entre eux, liés ou non entre eux, sont choisis parmi les groupements alkyles cycliques ou non, substitués ou non et contenant ou non des hétéroéléments, les groupements aromatiques, substitués ou non et contenant ou non des hétéroéléments,
- le groupement R² est choisi parmi les groupements alkyles cycliques ou non, substitués ou non et contenant ou non des hétéroéléments, les groupements aromatiques substitués ou non et contenant ou non des hétéroéléments,
- L² représente une base de Lewis,
- X¹ est un atome d'hydrogène ou un halogène ou un atome de carbone lié à ou faisant partie d'au moins un groupement alkyle cyclique ou non, insaturé ou non, substitué ou non et contenant ou non des hétéroéléments, un groupement aromatique substitué ou non et contenant ou non des hétéroéléments,
- L² et X¹ sont tels que le degré d'oxydation du nickel est respecté.

2. Complexe selon la revendication 1 dans lequel L² représente une phosphine de type P(A¹R'^{1a})(A'¹R'^{1b})(A"¹R'^{1c}) ou une phosphinamine de type (R'^{1a}A¹)(R'^{1b}A'¹)P-NH(R'²) ou (R'^{1a}A1)(R'^{1b}A'¹)P-NH-S(O)₂(R'²), dans lesquels :
- A¹, A'¹ et A"¹, identiques ou différents entre eux, sont indépendamment O, S, NR³, ou une liaison simple entre l'atome de phosphore et un atome de carbone,
- le groupement R³ est soit un atome d'hydrogène, soit un groupement alkyle cyclique ou non, substitué ou non et contenant ou non des hétéroéléments, ou un groupement aromatique, substitué ou non et contenant ou non des hétéroéléments,
- les groupements R'¹, soit R^{'1a}, R^{'1b} et R^{'1c} étant identiques ou différents entre eux, liés ou non entre eux, sont choisis parmi les groupements alkyles cycliques ou non, substitués ou non et contenant ou non des hétéroéléments, les groupements aromatiques, substitués ou non et contenant ou non des hétéroéléments,
- le groupement R'² est choisi parmi les groupements alkyles cycliques ou non, substitués ou non et contenant ou non des hétéroéléments, les groupements aromatiques substitués ou non et contenant ou non des hétéroéléments.

3. Complexe selon la revendication 1 ou 2 dans lequel les groupements R¹, soit R^{1a} et R^{1b} étant identiques ou différents, liés ou non entre eux, et les groupements R'¹, soit R'^{1a}, R^{'1b} et R^{'1c} étant identiques ou différents, liés ou non entre eux, sont choisis indépendamment parmi les groupements alkyles comprenant 1 à 15 atomes de carbones, les groupements aromatiques comprenant 5 à 20 atomes de carbone; substitués ou non, contenant des hétéroéléments ou non.

4. Complexe selon la revendication 3 dans lequel les groupements R¹, soit R^{1a} et R^{1b} étant identiques ou différents, liés ou non entre eux, et les groupements R'¹, soit R^{'1a}, R^{'1b} et R^{'1c} étant identiques ou différents, liés ou non entre eux, sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant ou non des hétéroéléments; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-di(trifluorométhyl)phényle, benzyle, naphthyle, bisnaphthyle, pyridyle, bisphényle, furanyle, thiophényle, substitués ou non, contenant des hétéroéléments ou non.

5. Complexe selon l'une des revendications 1 à 4 dans lequel les groupements R² et les groupements R'², identiques ou différents, sont indépendamment choisis parmi les groupements alkyles comprenant 1 à 15 atomes de carbones, les groupements aromatiques comprenant 5 à 20 atomes de carbone; substitués ou non, contenant des hétéroéléments ou non.

6. Complexe selon la revendication 5 dans lequel les groupements R² et les groupements R'², identiques ou différents, sont indépendamment choisis parmi les groupements méthyle, trifluorométhyle, éthyle, n-propyle, i-propyle, n-butyle, i-butyle, t-butyle, pentyle, cyclohexyle, adamantyle, substitués ou non, contenant des hétéroéléments ou non ; les groupements phényle, o-tolyle, m-tolyle, p-tolyle, mésityle, 3,5-diméthylphényle, 4-n-butylephényle, 4-méthoxyphényle, 2-méthoxyphényle, 3-méthoxyphényle, 4-méthoxyphényle, 2-isopropoxyphényle, 4-méthoxy-3,5-diméthylphényle, 3,5-ditert-butyl-4-méthoxyphényle, 4-chlorophenyle, 3,5-bis(trifluorométhyl)phényle, benzyle, naphthyle, bisnaphthyle, pyridyle, bisphényle, furanyle, thiophényle, substitués ou non, contenant des hétéroéléments ou non.

7. Procédé de préparation d'un complexe selon l'une des revendications 1 à 6 comprenant la mise en contact d'au moins un ligand comprenant ledit fragment de ligand constitué par les atomes P, N, S et O, avec au moins un précurseur de nickel de degré d'oxydation (0), un précurseur du groupement X¹, et optionnellement une base de Lewis.

8. Procédé selon la revendication 7 dans lequel le précurseur de nickel est choisi parmi le nickel(0) bis(cycloocta-1,5-diène), le nickel(0) bis(cycloocta-1,3-diène), le nickel(0) bis(cyclooctatétraène), le nickel(0) bis(cycloocta-1,3,7-triène), le bis(o-tolylphosphito)nickel(0)(éthylène), le nickel(0) tétrakis (triphénylphosphite), le nickel(0) tetrakis(triphenylphosphine), et le nickel (0) bis(éthylène), pris seul ou en mélange.

9. Procédé de préparation d'un complexe selon l'une des revendications 1 à 6 comprenant la mise en contact d'au moins un ligand comprenant ledit fragment de ligand constitué par les atomes P, N, S et O, avec au moins un précurseur de nickel de degré d'oxydation (+II) en présence d'un agent réducteur ou d'une base de Brönsted, et optionnellement une base de Lewis.

10. Procédé selon la revendication 9 dans lequel le précurseur de nickel est choisi parmi le chlorure de nickel(II), le chlorure de nickel(II)(diméthoxyéthane), le bromure de nickel(II), le bromure de nickel(II)(diméthoxyéthane), le fluorure de nickel(II), l'iodure de nickel(II), le sulfate de nickel(II), le carbonate de nickel(II), le dimethylglyoxime de nickel(II), l'hydroxyde de nickel(II), l'hydroxyacétate de nickel(II), l'oxalate de nickel(II), les carboxylates de nickel(II) tel que par exemple le 2-éthylhexanoate, les phénates de nickel(II), l'acétate de nickel(II), le trifluoroacétate de nickel(II), le triflate de nickel(II), l'acétylacétonate de nickel(II), l'hexafluoroacétylacétonate de nickel(II), le chlorure de allylnickel(II), le bromure de allylnickel(II), le dimère du chlorure de methallylnickel(II), l'hexafluorophosphate de allylnickel(II), l'hexafluorophosphate de methallylnickel(II), le biscyclopentadienyle de nickel(II), le bisallyl de nickel(II) et le bisméthallyl nickel(II); sous leur forme hydratée ou non, pris seul ou en mélange.

11. Utilisation d'un complexe selon l'une des revendications 1 à 6 ou préparé selon l'une des revendications 7 à 10 en tant que catalyseur.

12. Procédé d'oligomérisation d'une charge d'oléfines comprenant la mise en contact de ladite charge avec un complexe selon l'une des revendications 1 à 6 ou préparé selon les revendications 7 à 10, en présence ou pas de solvant.

13. Procédé selon la revendication 12 dans lequel le complexe est utilisé en mélange avec un composé choisi dans le groupe formé par les composés tris(hydrocarbyl)aluminium, les composés chlorés ou bromés d'hydrocarbylaluminium, les aluminoxanes, les composés organo-borés, les composés organiques susceptibles de donner ou capter un proton, pris seuls ou en mélange.

14. Procédé selon la revendication 12 ou 13 dans lequel la charge comprend des oléfines ayant un nombre d'atomes de carbone compris entre 2 et 10.

15. Procédé selon l'une des revendications 12 à 14 dans lequel la réaction est une réaction d'oligomérisation de l'éthylène.
